# EUROPEAN PATENT APPLICATION

(11) **EP 2 198 889 A1**
(43) Date of publication of application: **23.06.2010**
(21) Application number: 08382083.7
(22) Date of filing: 22.12.2008
(51) Int. Cl.: A61K 47/26, A61K 31/4045

(54) **Liquid pharmaceutical compositions comprising a triptan compound and Xylitol**

(71) Applicant: Almirall, S.A., 08022 Barcelona (ES)
(72) Inventor: Duarte Lopez, Begoña, 08301, MATARO (Barcelona) (ES); Guiro Coll, Pere, 08030, Barcelona (ES); Masso Carbonell, Antoni, 08800, VILANOVA I LA GELTRÚ, (Barcelona) (ES)
(74) Representative: Elzaburu Marquez, Alberto

(57) **Abstract**

An aqueous liquid pharmaceutical composition comprising a) at least one triptan compound, pharmaceutically acceptable salts or hydrates thereof, and b) xylitol.

## Description

### FIELD OF THE INVENTION

The present invention relates to aqueous liquid pharmaceutical compositions comprising at least one triptan compound and xylitol. Said compositions do not exhibit the bitter taste inherent to the triptan compounds and are remarkable more stable than compositions comprising other polyhydric alcohols. The invention further relates to a process for the preparation of said compositions and to methods of treatment by administering the compositions.

### BACKGROUND OF THE INVENTION

Migraine is a type of headache, which is a severe, seriously debilitating and usually unilateral form of episodic headache that may be preceded by aura and that is frequently associated with both neurological and gastrointestinal symptoms such as nausea, vomiting, diarrhea, sensitivity to light (photophobia), sound (phonophobia), and smells (osmophobia); sleep disruption, and depression. When untreated, a migraine headache attack may last anywhere from four to 72 hours.

Current migraine treatments are classified as prophylactic treatments or as abortive treatments. Each class of treatments is administered to the migraine sufferer based on the frequency and severity of the headache and its associated symptoms. Prophylactic treatments reduce the frequency of migraines and include non-steroidal antiinflammatory agents, andrenergic beta-blockers, calcium channel blockers, tricyclic antidepressants, selective serotonin reuptake inhibitors, or anticonvulsants. For occasional migraines, abortive treatments are used. The abortive treatments eliminate or reduce the severity of the headache and any associated symptoms after the migraine has begun. Abortive treatments include serotonin receptor agonists or ergotamine-based compounds, or compounds that provide analgesic effects.

"Triptans" or "Triptan compounds" are a family of tryptamine derivatives typically used as abortive medication in the treatment of acute attacks of migraine and cluster headaches. Generally, their action is attributed to their binding to serotonin 5-HT1 B and 5-HT1 D receptors in cranial blood vessels (causing their constriction) and subsequent inhibition of pro-inflammatory neuropeptide release.

Orally administered pharmaceutical compositions are provided to the patient in many dosage forms, including solid forms such as capsules (hard or soft) or tablets (uncoated or coated), and liquid forms such as solutions, emulsions or suspensions. Pharmaceutical compositions administered in solid form are usually intended to be swallowed whole.

Children, the elderly, and many other persons, including disabled or incapacitated patients, often have trouble swallowing tablets or capsules. In these situations, it is desirable to provide the drug either in a chewable solid form or in a liquid form. For many patients, including paediatric and geriatric patients, a liquid oral dosage form is preferable to a chewable dosage form, because of the ease with which it may be swallowed. Additionally, patients may be more inclined to comply with their medication instruction if the dosages are easier to ingest.

A common problem associated with oral liquid dosage forms is, as in the case of the triptans, the often disagreeable taste of the API contained therein. The unpleasant or bitter taste of the triptans does not need to be considered when formulating swallowable tablets or capsules, because these dosage forms are in the mouth such a short time that the pharmaceutical's taste can easily be masked with an exterior coating on the tablet. In the case of oral liquid formulations the disagreeable taste of the triptans has to be masked by adding sweeteners to the composition. However, these agents are not always totally effective in concealing the unpalatable taste of APIs.

However, some APIs like the triptans, which are unstable in front of light and/or oxidation, are especially unstable when present in liquid pharmaceutical compositions which need to be stored at long term and in adverse conditions of temperature and humidity, as recommended in ICH stability guidelines.

Surprisingly, we have now found that xylitol stabilizes liquid compositions comprising triptans and is therefore the most suitable sweetening agent. Furthermore, the presence of xylitol makes it unnecessary to use any other of the common excipients used in liquid compositions such as wetting agents, solubilizing agents, thickening agents, colorant agents, buffering agents and, in particular, preservative agents. This makes these compositions useful not only for oral administration, but also for parenteral administration.

Xylitol has the structure:

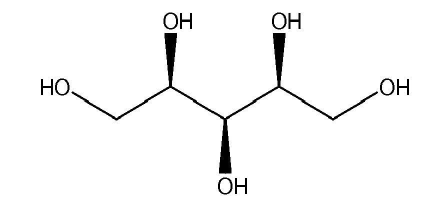

It is known as a possible pharmaceutical excipient (see for example WO-A-2007/070504).

### SUMMARY OF THE INVENTION

It has now surprisingly been found that an aqueous liquid pharmaceutical composition comprising: a) at least one triptan compound, or a pharmaceutically acceptable salt or hydrate thereof and b) xylitol, is stable against light and/or oxidation, and has an agreeable taste and is thus easy to swallow

The present invention further relates to a process for the preparation of the compositions as defined above which comprises the steps of:
a. dissolving an amount of at least one triptan compound, pharmaceutically acceptable salts or hydrates thereof in water or in a solution comprising essentially water to form an aqueous liquid triptan composition and,
b. combining xylitol with the aqueous liquid triptan composition and, optionally, with a flavouring agent, to form the aqueous liquid pharmaceutical composition.

The invention further relates to a composition as defined above for use in the treatment or prevention of migraine and/or cluster headache.

The invention further relates to use of a composition as defined above for the manufacture of a medicament for the treatment or prevention of migraine and/or cluster headache.

The invention further relates to a method for treating a subject afflicted with migraine and/or cluster headache, which comprises administering to said subject an effective amount of an aqueous liquid pharmaceutical composition as defined above.

Also provided is the use of xylitol to improve the stability of a triptan.

### DETAILED DESCRIPTION OF THE INVENTION

### The triptan compound

"Triptan" or "triptan compound" refers to a family of tryptamine derivatives typically used as abortive medication in the treatment of migraine and cluster headaches. Generally, their action is attributed to their binding to serotonin 5-HT1 B and 5-HT1 D receptors in cranial blood vessels (causing their constriction) and subsequent inhibition of pro-inflammatory neuropeptide release.

As such, triptan compound includes any tryptamine derivative providing such an effect described above. However, preferred triptan compounds can be selected from the group consisting of sumatriptan (3-[2-(dimethylamino)ethyl]-N-methyl-1H-indole-5-ylmethylsulfonamide), eletriptan (5-[2-(Benzenesulfonyl)ethyl]-3-[1-methylpyrrolidin-2(R)-ylmethyl]-1H-indole), rizatriptan (3-[2-(Dimethylamino)ethyl]-5-(1,2,4-triazol-1-ylmethyl)-1H-indole), frovatriptan ((R)-(+)-3-(Methylamino)-1,2,3,4-tetrahydro-9H-carbazole-6-carboxamide), almotriptan (3-[2-(Dimethylamino)ethyl]-5-(pyrrolidin-1-ylsulfonylmethyl)-1H-indole), zolmitriptan (4(S)-[3-[2-(Dimethylamino)ethyl]-1H-indol-5-ylmethyl]oxazolidin-2-one), naratriptan (N-Methyl-2-[3-(1-methylpiperidin-4-yl)-1H-indol-5-yl]ethanesulfonamide), pharmaceutically acceptable salts or hydrates thereof, and mixtures thereof. More preferred triptan compounds are sumatriptan, almotriptan, pharmaceutically acceptable salts or hydrates thereof, and mixtures thereof. The most preferred triptan compound according to the invention is almotriptan and pharmaceutically acceptable salts or hydrates thereof.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, benzoate, methanesulphonate and p-toluenesulphonate.

Preferred pharmaceutically acceptable salts or hydrates of the preferred triptan compounds can be selected from the group consisting of sumatriptan succinate, eleptritan hemisuccinate, eleptritan monohydrobromide, eleptritan monofumarate, eleptritan monosuccinate, rizatriptan sulphate, rizatriptan benzoate, frovatriptan succinate, almotriptan malate, zolmitriptan monohydrochloride, naratriptan monohydrochloride or mixtures thereof. More preferred pharmaceutically acceptable salts or hydrates of the preferred triptan compounds are sumatriptan succinate, almotriptan D,L hydrogen malate and mixtures thereof. Almotriptan D,L hydrogen malate is most preferred.

### Xylitol

According to the Handbook of Pharmaceutical Excipients, 5th edition, 2005 (ISBN 1582120587) edited by Pharmaceutical Press, xylitol (xylo-Pentane-1,2,3,4,5-pentol) occurs as a white, granular solid comprising crystalline equidimensional particles having a mean diameter of about 0.4-0.6 mm. It is odourless, with a sweet taste that imparts a cooling sensation.

As xylitol has an equal sweetness intensity to sucrose, combined with a distinct cooling effect upon dissolution of the crystal, it is highly effective at enhancing the flavour of tables and syrups and masking the unpleasant or bitter taste associated with triptans.

According to the invention, xylitol preferably has a mean particle size in the range of 90 to 700 µm, more preferably in the range of 150 to 350 µm.

### The aqueous liquid pharmaceutical compositions

The present invention provides an aqueous liquid pharmaceutical composition comprising
a) at least one triptan compound, preferably selected from the group consisting of sumatriptan, naratriptan, zolmitriptan, rizatriptan, almotriptan, eletriptan, frovatriptan, pharmaceutically acceptable salts or hydrates thereof (as described before), and mixtures thereof, and
b) xylitol.

According to the invention it is preferred that the triptan compound is almotriptan, pharmaceutically acceptable salts or hydrates thereof. Almotriptan D,L hydrogen malate is particularly preferred.

In the aqueous liquid pharmaceutical compositions according to the invention the total amount of a) the triptan compound is preferably in the range of 0.01 to 1 wt.%, more preferably 0.05 to 0.5 wt.% based on the total weight of the composition.
The total amount of b) xylitol is preferably in the range of 10 to 60 wt.%, more preferably 25 to 55 wt.%, most preferably 30 to 50 wt.%, based on the total weight of the composition.

Preferred aqueous liquid pharmaceutical compositions of the invention comprise, based on the total weight of the composition,
i) 0.05 to 0.5 wt.%, preferably 0.06 to 0.3 wt.% of a) at least one triptan compound, preferably almotriptan, pharmaceutically acceptable salts or hydrates thereof, more preferably almotriptan malate,
ii) 25 to 55 wt.%, preferably 30 to 50 wt.% of b) xylitol,
iii) balance water up to 100 wt.%.

The amount of each triptan compound which is required to achieve a therapeutic effect will, of course, vary with the particular active, the route of administration and the subject under treatment.

To provide a therapeutically effective amount of the triptan compound, preferably almotriptan, expressed as its base (not in salt form), it is generally present in an amount of 0.3 mg/mL to 15 mg/mL of the composition, preferably 0.35 mg/mL to 10 mg/mL, and more preferably 0.4 mg/mL to 5 mg/mL. The concentrations expressed herein are based on equivalents of triptan base.

The phrase "therapeutically effective amount" means that amount of the triptan compound, preferably almotriptan, that provides a therapeutic benefit in the treatment or management of migraine and/or cluster headache and general malaise associated therewith.

The aqueous liquid pharmaceutical compositions according to the invention optionally further comprise c) a flavouring agent. A "flavouring agent" as used herein is a substance capable of enhancing taste or aroma of the aqueous liquid pharmaceutical compositions according to the invention, making then more pleasant. Suitable flavouring agents can be selected from standard reference books, for example Fenaroli's Handbook of Flavor Ingredients, 5th edition (2004).

Flavouring agents may include natural flavouring agents, nature-identical flavouring agents, artificial flavouring agents, or mixtures thereof.
Natural flavouring substances are flavouring substances obtained from plant or animal raw materials, by physical, microbiological or enzymatic processes. They can be either used in their natural state or processed for human consumption. Natural flavouring agents may include, but not limited to, oils derived from plants and fruits such as peppermint oils, spearmint oils, mentha arvensis oils, aniseed oils, clove oils, citrus oils, cinnamon bark oils, strawberry oils, tangerine oils, orange oils, winter green oils, cassia oils, davana oils, spruce needle oils, eucalyptus oils, fennel oils, galbanum oils, ginger oils, camomile oils, cumin oils, rose oils, geranium oils, sage oils, yarrow oils, star anise oils, thyme oils, juniper berry oils, rosemary oils, angelica root oils, and fractions of these oils. Among natural flavouring agents it can also be mentioned anethole, caraway, cardamom seed, cherry juice, cherry syrup, cinnamon, citric acid, cocoa, ginger, glycyrrhiza, honey, orange syrup, peppermint, raspberry juice, spearmint, vanilla and vanillin.

Nature-identical flavouring agents are flavouring substances that are obtained by synthesis or isolated through chemical processes, which are chemically identical to flavouring substances naturally present in products intended for human consumption. Artificial flavouring agents are flavouring substances not identified in a natural product intended for human consumption, whether or not the product is processed.

Flavouring agents can be used singly or in combinations of two or more. Additionally, natural, nature-identical and artificial flavouring agents may be combined in any sensorially acceptable fashion.

Flavouring agents can be used in powder form, in the form of liquid preparations, in the form of suspensions, in the form of elixirs or in the form of syrups containing said flavouring agents.

Flavouring agents can be combined with aroma substances. Examples of homogeneous aroma substances are, for example: anethole, menthol, menthone, isomenthone, menthyl acetate, menthofuran, mint lactone, eucalyptol, limonene, eugenol, pinene, sabinene hydrate, 3-octanol, carvone, gamma-octalactone, gamma-nonalactone, germacrene-D, viridiflorol, 1,3E,5Z-undecatriene, isopulegol, piperitone, 2-butanone, ethyl formate, 3-octyl acetate, isoamyl isovalerate, hexanol, hexanal, cis-3-hexenol, linalool, alpha-terpineol, cis and trans carvyl acetate, p-cymol, damascenon, damascone, rose oxide, dimethyl sulfide, fenchol, acetaldehyde diethyl acetal, cis-4-heptenal, isobutyraldehyde, isovaleraldehyde, cis-jasmone, anisaldehyde, methyl salicylate, myrtenyl acetate, 2-phenylethyl alcohol, 2-phenylethyl isobutyrate, 2-phenylethyl isovalerate, cinnamaldehyde, geraniol, nerol. In the case of chiral compounds, the aroma substances can be present as racemate or an individual enantiomer or as enantiomer-enriched mixture.

Flavouring may include a cooling agent to enhance the flavor and perceived breath freshening of the product. Cooling agents include menthol, ethyl p-menthane carboxamide, N,2,3-trimethyl-2-isopryl-butanamide, menthyl glutarate, menthyl succinate, menthol PG carbonate, menthol EG carbonate, menthyl lactate, menthone glyceryl ketal, menthol glyceryl ether, N-tertbutyl-p-menthane-3-carboxamide, p-menthane-3-carboxylic acid glycerol ester, methyl-2-isopryl-bicyclo, heptane-2-carboxamide, menthol methyl ether and combinations thereof.

The pH value of the aqueous liquid pharmaceutical compositions according to the invention is typically within the acceptable range for physiological administration, and is preferably in the range of 3.0 to 7.0, more preferably in the range of 4 to 6.

The aqueous liquid pharmaceutical compositions according to the invention may optionally further comprise sweetening agents other than xylitol, wetting agents, solubilizing agents, thickening agents, colorant agents, preservative agents, buffering agents or mixtures thereof.

Examples of sweetening agents other than xylitol include sugar sweeteners such as monosaccharides, disaccharides and polysaccharides (xylose, ribose, glucose, mannose, galactose, fructose, dextrose, sucrose, maltose, partially hydrolyzed starch (such as maltitol syrup) or corn syrup solids); sugar alcohols (sorbitol, mannitol); other polyols (glycerine, propylene glycol) optionally alkoxylated with ethylene oxide or popylene oxide; artificial sweeteners such as the soluble saccharin salts, i. e., sodium, or calcium saccharin salts, cyclamate salts, acesulfam-K, ammonium glycyrrhizinate, dipotassium glycyrrhizinate and the like, and the free acid form of saccharin; and dipeptide based sweeteners such as L-aspartylphenylalanine methyl ester.

Examples of wetting agents (chemical substances that increase the spreading and penetrating properties of a liquid by lowering its surface tension) include one or more cationic surfactants, such as benzalkonium chloride; non-ionic surfactants such as polyoxyethylene and polyoxypropylene block copolymers; polyoxyethylene fatty acid glycerides and oils (such as polyoxyethylene (6) caprylic/capric mono- and diglycerides), polyoxyethylene (40) hydrogenated castor oil; polyoxyethylene sorbitan esters, such as polysorbate 20 and polysorbate 80; propylene glycol fatty acid esters, such as propylene glycol laureate; glyceryl fatty acid esters, such as glyceryl monostearate; sorbitan esters, such as sorbitan monolaurate, sorbitan monooleate, sorbitan monopalmitate and sorbitan monostearate; glyceryl fatty acid esters, for example glyceryl monostearate; or anionic surfactants such as sodium lauryl sulphate, sodium lauryl ether sulphate; fatty acids and salts thereof, such as oleic acid, sodium oleate and triethanolamine oleate.

Examples of solubilizing agents are, for example, nonionic surfactants from at least one of the following groups: products of the addition of 1 to 30 moles of ethylene oxide and/or 0 to 5 moles of propylene oxide onto linear C₈-C₂₂ fatty alcohols, C₁₂-C₂₂ fatty acids and alkyl phenols containing 8 to 15 carbons in the alkyl group; alkyl and/or alkenyl oligoglycosides containing 8 to 22 carbons in the alkyl group and ethoxylated analogs thereof; addition products of 1 to 15 moles of ethylene oxide with castor oil and/or hydrogenated castor oil; addition products of 15 to 60 moles of ethylene oxide with castor oil and/or hydrogenated castor oil; partial esters of glycerol and/or sorbitan with unsaturated or saturated, linear or branched fatty acids containing 12 to 22 carbons and/or hydroxycarboxylic acids containing 3 to 18 carbon atoms and addition products thereof with 1 to 30 moles of ethylene oxide; mixtures of alkoxylated glycerides and alkoxylated glycerine, partial esters of polyglycerol (average degree of selfcondensation 2 to 8), polyethylene glycol (weight average molecular weight 400 to 5000), trimethylolpropane, pentaerythritol, sugar alcohols (for example sorbitol), alkyl glucosides (for example methyl glucoside, butyl glucoside, lauryl glucoside) and polyglucosides (for example cellulose) with saturated and/or unsaturated, linear or branched fatty acids containing 12 to 22 carbons and/or hydroxycarboxylic acids containing 3 to 18 carbons and addition products thereof with 1 to 30 moles of ethylene oxide; mixed esters of pentaerythritol, fatty acids, citric acid and fatty alcohol and/or mixed esters of fatty acids containing 6 to 22 carbons, methyl glucose and polyols, preferably glycerol or polyglycerol; mono-, di- and trialkyl phosphates and mono-, di- and/or tri-PEG-alkyl phosphates and salts thereof; block copolymers, for example Polyethyleneglycol-30 Dipolyhydroxystearate; polymer emulsifiers; polyalkylene glycols and alkyl glyceryl ethers.

A thickening agent or viscosity-enhancing agent can be included to generally thicken the liquid pharmaceutical compositions. While any suitable thickening agent can be included in the compositions of the present invention, a preferred thickening agent, when used, includes one or more of acacia, alginic acid bentonite, carbomer, carboxymethylcellulose calcium or sodium, cetostearyl alcohol, methyl cellulose, ethylcellulose, glycerin, gelatin guar gum, hydroxyethylcellulose, hydroxypropylcellulose, hydroxypropylmethylcellulose, maltodextrin, polyvinyl alcohol, povidone, propylene carbonate, propylene glycol alginate, sodium alginate, sodium starch glycolate, starch tragacanth, and xanthan gum, and any combination thereof. More preferred thickening agents are glycerin, hydroxypropylmethylcellulose, and xanthan gum, and any combination thereof.

Optionally, the aqueous liquid pharmaceutical compositions according to the invention further comprise a colouring agent. Suitable colouring agents illustratively include FD & C Red No. 3, FD & C Red No. 20, FD & C Red No. 40, FD & C Yellow No. 6, FD & C Blue No. 2, D & C Green No. 5, D & C Orange No. 5. A full recitation of all FD & C and D & C and their corresponding chemical structures may be found in the Kirk Othmer Encyclopedia of Chemical Technology.

Examples of suitable preservatives to prevent microbial contamination are alkylparabens, particularly methylparaben, propylparaben and butylparaben; sodium benzoate; butylated hydroxy toluene; butylated hydroxyanisole; ethylenediamine tetraacetic acid; chlorobutanol; benzyl alcohol; phenylethylalcohol; dehydroacetic acid; sorbic acid; potassium sorbate; benzalkonium chloride; benzethonium chloride; and mixtures thereof. The amount of preservative generally utilized will vary depending upon the preservative selected.

Preferably, the aqueous liquid pharmaceutical compositions according to the invention do not need any preservative to prevent microbiological contamination, i.e. are preservative free.

Any pharmaceutically acceptable buffering agents to adjust the pH of the aqueous liquid pharmaceutical compositions according to the invention to be within the acceptable range for physiological administration, preferably in the range of 3.0 to 7.0, more preferably in the range of 4 to 6, can be used. For example inclusion in the composition of a pharmaceutically acceptable acid such as acetic, citric, fumaric, phosphoric, hydrochloric, lactic or nitric acids or the like, or a mixture thereof. It will also be understood that certain compositions of the invention can have a pH in the desired range without inclusion of a pH adjusting agent specifically for that purpose. Typically, however, an acidic buffer system is present in the composition to achieve the desired pH. An acidic buffer system comprises an acidulant and a buffering agent. Suitable acidulants will be known to those of skill in the art and illustratively include acetic, citric, fumaric, hydrochloric, phosphoric, lactic and nitric acids and the like, and mixtures thereof. Suitable buffering agents will likewise be known to those of skill in the art and illustratively include potassium metaphosphate, potassium phosphate, sodium phosphate, sodium acetate, sodium citrate and the like, and mixtures thereof.

According to the invention, there is no need to add to the liquid pharmaceutical compositions according to the invention a buffering agent in order to improve the stability.

It is also an object of the present invention a process for the preparation of the aqueous liquid pharmaceutical compositions described before, which comprises the steps of:
a. dissolving an amount of at least one triptan compound (preferably almotriptan), pharmaceutically acceptable salts or hydrates thereof into water or into a solution comprising essentially water to form an aqueous liquid triptan composition and,
b. combining xylitol with the aqueous liquid triptan composition and, optionally, with the flavouring agent, to form the aqueous liquid pharmaceutical composition.

When the flavouring agent is present, it may be added before or after combining xylitol with the aqueous liquid triptan composition.

According to the invention, xylitol can be used either in solid form or in the form of a solution.

The phrase "a solution comprising essentially water" in step a) means that water is the essential ingredient of the solution, but it may contain optionally other non-essential ingredients for the purpose of the present invention selected from the ingredients well known to the person skilled in the art.

A method for treating a subject afflicted with migraine and/or cluster headache, which comprises administering to said subject an effective amount of an aqueous liquid pharmaceutical compositions according to the invention also forms part of the object of the present invention.

As used herein, the term subject refers to a mammal that may benefit from the administration of a drug composition or method of this invention. Preferably, subject refers to humans.

The invention further relates to the composition of the invention for use in the treatment of the human or animal body, preferably for use in the treatment or prevention of migraine and/or cluster headache. Thus, the invention further relates to use of a composition as defined above for the manufacture of a medicament for the treatment or prevention of migraine and/or cluster headache.

The present invention also provides the use of xylitol to improve the stability of a triptan, in particular almotriptan. Preferably, the xylitol is used to reduce the oxidation of the triptan caused by heat and/or light. Typically, the xylitol is used to improve the stability of a triptan in an aqueous liquid pharmaceutical composition as defined above.

The aqueous liquid pharmaceutical compositions of the present invention may be administered by any suitable route, e.g. orally (as solutions, suspensions and emulsions, etc); or by parenteral injection (subcutaneous, intradermic, intramuscular, intravenous, etc.) For parenteral injections, aqueous liquid pharmaceutical compositions can be formulated with polyethyelene glycols. However, the preferred route of administration of the aqueous liquid pharmaceutical compositions of the present invention is oral.

The aqueous liquid pharmaceutical compositions of the present invention may be packaged in any suitable container, such as HDPE bottle packs, PET, or amber glass bottle packs, glass tubing, tubular vials, glass ampoules, thermo-form packaging, pouches, sachets and stick packs.

The pharmaceutical compositions may conveniently be presented in unit dosage form so that the patient may administer a single dose and may be prepared by any of the methods well known in the art of pharmacy. Preferably said unit dosage form ranges from 3 to 25 mL, more preferably from 5 to 20 mL, even more preferred from 6 to 15 mL.

The following examples are given in order to provide a person skilled in the art with a sufficiently clear and complete explanation of the present invention, but should not be considered as limiting of the essential aspects of its subject, as set out in the preceding portions of this description.

### EXAMPLES

### Example 1 - One week stress stability tests

The physical and chemical stability of aqueous liquid compositions of almotriptan malate in combination with different sweetening agents (Table 1) was evaluated using a stability screening test during one week at 50°C.
To prepare each composition, 17.5 mg of almotriptan D,L hydrogen malate were weighed and dissolved into purified water in a 10 mL tubular amber vial. To said mixture, the sweetener or mixture of sweeteners was added. After homogenization, said compositions were stored 1 week at 50°C.
The presence of almotriptan oxidation impurities was followed by High Performance Liquid Chromatography (HPLC) determination. Results are indicated in Table 1.

The total amount of oxidation impurities should be as lower as possible, preferably lower than 0.1 wt.%, with respect to the amount of almotriptan. Additionally, the amount of each individual impurity (impurity 1 and impurity 2) should be also as lower as possible.

Compositions 1 and 2 are according to the invention and compositions C1-C5 are comparative experiments.

**Table 1 - Stability of aqueous liquid compositions of almotriptan D,L hydrogen malate in combination with different sweetening agents stored one week at 50 °C**

| Composition | Sweetener (or mixture of sweeteners) | | Impurity 1¹ | Impurity 2¹ | Total¹ |
|---|---|---|---|---|---|
| | | | | | (1 +2) |
| C1 | Fructose | 50% | 5.60% | 3.66% | 9.26 |
| C2 | Sorbitol | 70% | 0.35% | 0.20% | 0.55 |
| C3 | Sorbitol | 45% | 0.42% | 0.30% | 0.72 |
| | Glycerin | 30% | | | |
| C4 | Sorbitol | 55% | 0.40% | 0.28% | 0.68 |
| | Glycerin | 20% | | | |
| C5 | Sorbitol | 25% | 0.34% | 0.20% | 0.54 |
| | Glycerin | 25% | | | |
| | Xylitol | 25% | | | |
| 1 | Xylitol | 30% | 0.06% | 0.03% | 0.09 |
| 2 | Xylitol | 50% | 0.05% | 0.02% | 0.07 |

| | | | | | |
|---|---|---|---|---|---|
| ¹wt.% with respect to the total weight of almotriptan | | | | | |

From the experimental results it can be concluded that the aqueous liquid pharmaceutical compositions according to the invention (Compositions 1 and 2) provide an improved chemical stability against oxidation of the triptan compounds, in particular almotriptan. Additionally, the compositions C1-C5 were brown coloured after the one week stability test.

Particularly good results were obtained when the xylitol content is 50 wt.% (Composition 2) based on the total weight of the aqueous liquid pharmaceutical composition.

Furthermore, the microbiology stability of Compositions 1 and 2 was checked. Said solutions had inherent preservation properties, especially Composition 2 and, therefore, no preservative agent is needed to prevent microbiology contamination.

It is a finding of the present invention that xylitol is effective in improving the stability of a triptan, in particular almotriptan. Preferred compositions of the invention are those which, when stored for one week at 50°C, contain a total amount of oxidation impurities (as determined by High Performance Liquid Chromatography (HPLC)) which is less than 0.1 wt.%, with respect to the amount of triptan.

### Example 2 - Six months accelerated stability test

The following composition, Composition 3, (9.5 g) was prepared and introduced into a stick pack (polyester 12 µm - Al 9 µm - polyethylene 50 µm):

| | |
|---|---|
| - Almotriptan D,L hydrogen malate | 17.5 mg |
| - Xylitol | 4750 mg |
| - Flavouring agent | s.q. |
| - Purified water | Added up to 100% |

To prepare the composition, 17.5 mg of almotriptan D,L hydrogen malate were weighed and dissolved into purified water. To said mixture, the flavouring agent was added. After homogenization, xylitol was added. Finally, after homogenization, said composition was stored six months at 40°C and 75% RH.
The presence of almotriptan oxidation impurities was followed by HPLC. Results are indicated in Table 2.

**Table 2 - Stability of aqueous liquid compositions of almotriptan D,L hydrogen malate in combination with xylitol stored six months at 40 °C and 75 % RH**

| | Time (months) | | | |
|---|---|---|---|---|
| Impurities (wt.%) | 0 | 2 | 4 | 6 |
| Impuritiy 1 | <0.01 % | 0.14% | 0.21% | 0.38% |
| Impuritiy 2 | <0.01 % | 0.06% | 0.08% | 0.14% |
| Total (1+2) | <0.01% | 0.20% | 0.29% | 0.52% |

From the experimental results it can be concluded that the aqueous liquid pharmaceutical compositions according to the invention (Composition 3) may provide a good chemical stability against oxidation of the triptan compounds even at long term storage conditions.

The microbiology stability and the organoleptic characteristics of said composition was also checked and it was under specifications.

## Claims

1. An aqueous liquid pharmaceutical composition comprising a) at least one triptan compound, or a pharmaceutically acceptable salt or hydrate thereof, and b) xylitol.

2. The composition according to claim 1, wherein the triptan compound is selected from the group consisting of sumatriptan, naratriptan, zolmitriptan, rizatriptan, almotriptan, eletriptan, frovatriptan, pharmaceutically acceptable salts or hydrates thereof, and mixtures thereof.

3. The composition according to claim 1 or 2, wherein the triptan compound is almotriptan, pharmaceutically acceptable salts or hydrates thereof.

4. The composition according to any one of claims 1 to 3, wherein the total amount of a) the triptan compound is in the range of 0.01 to 1 wt. % based on the total weight of the composition.

5. The composition according to claim 4, wherein the total amount of a) the triptan compound is in the range of 0.05 to 0.5 wt. % based on the total weight of the composition.

6. The composition according to any one of claims 1 to 5, wherein the total amount of b) xylitol is in the range of 10 to 60 wt. % based on the total weight of the composition.

7. The composition according to claim 6, wherein the total amount of b) xylitol is in the range of 25 to 55 wt. % based on the total weight of the composition.

8. The composition according to any one of claims 1 to 7 comprising, based on the total weight of the composition,
i) 0.05 to 0.5 wt. % of a) at least one triptan compound,
ii) 25 to 55 wt. % of b) xylitol,
iii) balance water up to 100 wt. %.

9. The composition according to claim 8 comprising, based on the total weight of the composition,
i) 0.05 to 0.5 wt% of almotriptan, pharmaceutically acceptable salts or hydrates thereof,
ii) 25 to 55 wt.% of b) xylitol,
iii) balance water up to 100 wt.%.

10. The composition according to any one of the preceding claims, further comprising c) a flavouring agent.

11. The composition according to any one of the preceding claims, wherein the composition has a pH value in the range of 3.0 to 7.0.

12. A process for the preparation of the compositions according to claims 1 to 11, which comprises the steps of:
a. dissolving an amount of at least one triptan compound, pharmaceutically acceptable salts or hydrates thereof into water or into a solution comprising essentially water to form an aqueous liquid triptan composition and,
b. combining xylitol with the aqueous liquid triptan composition and, optionally, with the flavouring agent, to form the aqueous liquid pharmaceutical composition.

13. A composition according to any one of claims 1 to 11 for use in the treatment or prevention of migraine and/or cluster headache.

14. Use of a composition according to any one of claims 1 to 11 for the manufacture of a medicament for the treatment or prevention of migraine and/or cluster headache.

15. A method for treating a subject afflicted with migraine and/or cluster headache, which comprises administering to said subject an effective amount of a composition as defined in any one of claims 1 to 11.

16. Use of xylitol to improve the stability of a triptan.
